# EUROPEAN PATENT SPECIFICATION

(11) **EP 3 171 873 B1**
(45) Date of publication and mention of the grant of the patent: **25.12.2019**
(21) Application number: 15720286.2
(22) Date of filing: 17.04.2015
(51) Int. Cl.: A61K 31/355, A61P 11/02, A61P 37/08, A61K 9/00, A61K 47/32, A61K 47/44, A61K 45/06

(54) **COMPOSITION COMPRISING AN ESTER OF ALPHA-TOCOPHEROL FOR PREVENTION AND TREATMENT OF ALLERGIC RHINITIS**
ZUSAMMENSETZUNG ENTHALTEND EINEN ESTER DES ALPHA-TOCOPHEROLS ZUR PRÄVENTION UND BEHANDLUNG VON ALLERGISCHER RHINITIS
COMPOSE A BASE D'UN ESTER DU ALPHA-TOCOPHEROL POUR LA PREVENTION ET LE TRAITEMENT DE LA RHINITE ALLERGIQUE

(30) Priority: 22.07.2014 IT MI20141332
(43) Date of publication of application: 31.05.2017
(73) Proprietor: BIO.LO.GA. S.r.l., 31015 Conegliano (TV) (IT)
(72) Inventor: PANIN, Giorgio, 45100 Rovigo (IT); LAMPRECHT, Jürgen, 45131 Essen (DE)
(74) Representative: Ferreccio, Rinaldo
(86) International application number: PCT/EP2015/058368
(87) International publication number: WO 2016/012110

(56) References cited:
- EP-A1- 1 094 807
- EP-A1- 2 233 124
- EP-A2- 2 334 186
- WO-A2-97/45098
- DE-A1- 4 327 089
- US-A- 4 269 835
- US-A- 4 364 945
- DATABASE WPI Week 200365 2003 Thomson Scientific, London, GB; AN 2003-683319 XP002737135, & JP 2003 128552 A (ROHTO SEIYAKU KK) 8 May 2003 (2003-05-08)
- DATABASE WPI Week 200877 2008 Thomson Scientific, London, GB; AN 2008-N11835 XP002737136, & JP 2008 266255 A (UNIV TOHOKU) 6 November 2008 (2008-11-06)
- DATABASE MEDLINE [Online] US NATIONAL LIBRARY OF MEDICINE (NLM), BETHESDA, MD, US; July 2013 (2013-07), LI YANQING ET AL: "Randomized double-blind placebo-controlled crossover study of efficacy of pollen blocker cream for perennial allergic rhinitis.", XP002737137, Database accession no. NLM23883812 & AMERICAN JOURNAL OF RHINOLOGY & ALLERGY 2013 JUL-AUG, vol. 27, no. 4, July 2013 (2013-07), pages 299-303, ISSN: 1945-8932
- TANJA HILDENBRAND ET AL: "Rhinitis sicca, dry nose and atrophic rhinitis: a review of the literature", EUROPEAN ARCHIVES OF OTO-RHINO-LARYNGOLOGY ; AND HEAD & NECK, SPRINGER, BERLIN, DE, vol. 268, no. 1, 29 September 2010 (2010-09-29), pages 17-26, XP019869962, ISSN: 1434-4726, DOI: 10.1007/S00405-010-1391-Z

## Description

### Field of application

The present invention relates to the field of the pharmaceutical industry. In particular, the invention relates to a pharmaceutical composition for use in the prevention and treatment of allergic rhinitis.

### Prior Art

Allergic rhinitis is a widespread disease in Europe and in the western countries in general. In case of local exposure of the nasal mucosa to an allergen, a reaction mediated by IgE leads to nasal obstruction, hypersecretion and sneezing attacks, mostly related also to allergic conjunctivitis, disorders of the sleep and the general state of health.

For the treatment of allergic rhinitis it is advised to avoid or reduce exposure to allergens and take medication for topical or systemic use, especially antihistamines and corticosteroids. The side effects of these drugs, such as fatigue and propensity for atrophic rhinitis, are quite rare and must anyway be taken into consideration.

The patient compliance to therapy is, however, limited and inadequate treatment leads to the risk of a "horizontal" diffusion of the disease in relation to other allergens and a "vertical" diffusion in the direction of allergic asthma.

It would therefore be desirable to have alternative therapeutic approaches that would ensure good adherence of patients to treatment, thus avoiding the aforementioned risks of horizontal and vertical diffusion of atrophic rhinitis.

Patent application WO 2004/045556 discloses a method for preventing or treating allergic inflammation in a patient, comprising administering to the patient a compound that inhibits reactive oxygen species.

In the above-mentioned patent application, by the term "allergic inflammation" several diseases including allergic inflammation of the epithelium of the respiratory tract and mucous membranes of the nose are meant.

The compound in question may be a NADH / NADPH oxidase inhibitor, a superoxide dismutase inhibitor or a compound that inhibits the activity of reactive oxygen species. The latter compound can be, among others, tocopherol.

Patent application US 2010/0076072 relates to a method for treating inflammation, comprising the administration of tocopherol comprising at least 90% of alpha-tocopherol. No specific mention is made of a treatment of allergic rhinitis.

Patent application DE 43 27 089 A1 claims an agent for the external treatment of eyes, nose and throat in case of allergies, coughs and sneezes, as well as mild asthma conditions, which contains vitamin E optionally in combination with mediator release inhibitors and/or vitamin A, optionally with one or more compounds of the groups of vitamins of the B series, vitamin C, preservatives, emulsifiers, carriers and adjuvants.

Although the presence of mediator release inhibitors is claimed as optional, only agents containing such inhibitors are in fact described and exemplified in this application. Moreover, it is stated that the agents according to this invention have synergistic effects and that vitamin E surprisingly improves the efficacy and the absorption of cromoglycate and its derivatives, whereas no effects are shown or mentioned for agents in which vitamin E is the only active substance.

JP 2003128552 A discloses an aqueous eye drop composition comprising i.a. a xanthin compound, d,l-alpha-tocopheryl acetate and polysorbate 80, which can be used for the treatment of allergic rhinitis.

US 4 269 835 and US 4 364 945 disclose compositions for topical application to the nasal passageways for the treatment of hay fever and nasal allergy problems consisting essentially of a buffered, isotonic solution containing an antihistamine and a saturated amount of one or more of the natural and synthetic isomers of tocopherol or the esters of one of the isomers of tocopherol. These compositions can also include an emulsifier to aid in holding the vitamin E compound in solution.

### Summary of the Invention

The object of the present invention was to make available a new therapeutic approach for the prevention and treatment of allergic rhinitis, which is substantially free of side effects and that can ensure a good adherence of patients to therapy.

This object has been achieved by providing a a composition for topical application, for use in the prevention or treatment of allergic rhinitis, consisting of
an ester of alpha-tocopherol selected from the group consisting of tocopheryl acetate, n-propionate and linoleate, and
an oily vehicle selected from the group consisting of hydrogenated polyisobutene, hydrogenated polydecene, mixtures of hydrogenated polyisobutene and/or hydrogenated polydecene with hydrogenated polyolefins (in particular hydrogenated C₆-C₁₄ hydrogenated polyolefins), Caprylic/Capric Triglyceride, Olus Oil, Adansonia Digitata Oil, Adansonia Digitata Seed Oil, Coco-Caprylate/Caprate, Olive Squalane, Olive Squalene, Sunflower (Heliantus Annus) Seed Oil, Coco-Caprylate, Isononyl Isononanoate, Cyclopentasiloxane, and mixtures thereof.

Particularly preferred is the use of alpha-tocopheryl acetate.

Preferably the composition comprises from 3% to 80%, conveniently from 5 to 40%, advantageously from 10% to 30% by weight of said ester of tocopherol on the total weight of the composition.

A preferred vehicle is constituted by hydrogenated polydecene.

A particularly preferred composition consists of alpha-tocopheryl acetate and hydrogenated polydecene.

Advantageously, this composition consists of 10-30% alpha-tocopheryl acetate and 70-90% hydrogenated polydecene.

All percentages given in this application are to be understood, unless otherwise indicated, as percentages by weight of the total weight of the composition.

The invention is defined in the claims.

### Detailed description of a preferred embodiment

Patent applications WO 2004/045556 and US 2010/0076072 mentioned above had mentioned the usefulness of free tocopherol in the prevention and treatment of inflammation, including allergic inflammation, by virtue of its characteristics of antioxidant agent and reactive oxygen species inhibitor. These characteristics have been known for a long time and only belongs to free tocopherol and not to its esters (in which the hydroxyl on the aromatic ring is blocked) and in fact the two patent applications in question make no mention of the possibility of using tocopherol esters for the proposed treatment.

The Applicant has been producing for a number of years a preparation based on alpha-tocopheryl acetate, marketed under the name of VEA® Oris (14% alpha-tocopheryl acetate and 86% hydrogenated polydecene), for application to the oral-pharyngeal mucosa, having an emollient and protective action. In view of the favorable effects observed in the prevention and treatment of pharyngitis, which go beyond what might be expected from a simple emollient effect on the mucous membrane, and in view also of the above highlighted need for an alternative therapeutic approach for allergic rhinitis, the Applicant has thought to verify whether the above product shows an effect also on the nasal mucosa of patients with allergic rhinitis.

It was actually found, in a totally surprising way, if one considers that tocopheryl acetate does not exert the antioxidant action mentioned in the two documents of the prior art cited above, that the product VEA® Oris exerts a prophylactic and therapeutic action with respect to allergic rhinitis, as will be evident from the experimental results described below. In the period from the end of February to early May (season of the dispersal of pollen of early blooming plants, especially birch), 17 patients with allergic rhinitis with a clear anamnestic demonstration were treated with VEA® Oris by application onto the nasal mucosa. Patients were allowed to maintain, reduce or suspend use of antihistamines (loratadine or desloratadine) and topical mometasone, which had previously been largely successful.

### Methodology

Depending on the load of airborne pollen, VEA® Oris had to be applied from 4 to 6 times a day onto the nasal mucosa by spraying, according to the need and the effect. Next to allergic rhinitis, some patients had side diagnoses of diseases such as sinusitis (n = 7), upper respiratory tract infection (n = 8), disorders of the Eustachian tube (n = 6), respiratory disorders / snoring related to sleep (n = 9) and other. Seven patients had never been treated before for allergy, eight were dissatisfied with the pharmacological therapy so far adopted (cetrizine, loratadine, desloratadine, mometasone). Two were satisfied with the treatment for their allergy but showed themselves open to trying alternative therapies. All patients were recommended to avoid contact with allergens and were prescribed the classic therapy with an antihistamine and topical corticosteroid spray for the nose. In addition, they were asked to perform a test with the product VEA® Oris for local application in the nose.

### Results

All 17 patients performed the self treatment with VEA® Oris, sprayed into the nose from four to six times a day. Ten patients were examined at the end of treatment and the other seven were questioned by telephone.

All patients were convinced of the existence of a clear effect on nasal breathing, nasal secretion, sleep and overall quality of life in everyday life and in the workplace. All patients reported a reduction of the therapy with antihistamine and / or mometasone, five patients completely stopped the previously used therapy. All patients expressed their willingness to continue the use of VEA® Oris also in the next season of pollen dispersal.

An improvement of allergic conjunctivitis was not observed.

According to a not binding hypothesis, the favorable effect of VEA® Oris, that is of a composition of alpha-tocopheryl acetate and hydrogenated polydecene, on allergic rhinitis could be due to the formation of a film on the mucous membrane, which locally prevents the contact of the allergen - in this specific case birch pollen - with the sensitized mucosa. It is also conceivable that alpha-tocopheryl acetate might exert a chemical action on the surface proteins of pollen, which could weaken or nullify the aggressiveness of the allergen.

The following formulations were also prepared:
1)
   Alpha-tocopheryl linoleate 14%
   Hydrogenated polydecene 86%
2)
   Alpha-tocopheryl acetate 10%
   Hydrogenated polyisobutene 90%
3)
   Alpha-tocopheryl acetate 25%
   Olus Oil 75%

A preliminary trial of the three formulations reported above on three respective groups of five patients with a certain diagnosis of allergic rhinitis, in the same experimental conditions of the test reported above, with self-administration of the formulation by spraying into the nasal mucosa from 4 to 6 times a day for a period of 30 days gave results comparable to those obtained in the test with the product VEA® Oris.

## Claims

1. A composition for topical application, for use in the prevention or treatment of allergic rhinitis, consisting of
an ester of alpha-tocopherol selected from the group consisting of alpha-tocopheryl acetate, n-propionate and linoleate, and
an oily vehicle selected from the group consisting of
hydrogenated polyisobutene, hydrogenated polydecene,
mixtures of hydrogenated polyisobutene and/or hydrogenated polydecene with hydrogenated polyolefins, in particular hydrogenated C₆-C₁₄ hydrogenated polyolefins,
Caprylic/Capric Triglyceride, Olus Oil, Adansonia Digitata Oil, Adansonia Digitata Seed Oil, Coco-Caprylate/Caprate, Olive Squalane, Olive Squalene, Sunflower (Heliantus Annus) Seed Oil, Coco-Caprylate, Isononyl Isononanoate, Cyclopentasiloxane, and mixtures thereof.

2. The composition for the use of claim 1, wherein said composition comprises 3% to 80% by weight of said ester of alpha-tocopherol on the total weight of the composition.

3. The composition for the use of claim 1, wherein said composition comprises 5% to 40%, preferably 10% to 30% by weight of said ester of alpha-tocopherol on the total weight of the composition.

4. The composition for the use of any one of claims 1 to 3, wherein said ester of alpha-tocopherol is alpha-tocopheryl acetate.

5. The composition for the use according to any one of claims 1 to 4, wherein said oily vehicle consists of hydrogenated polydecene.

6. The composition for the use according to claim 5, wherein said ester of alpha-tocopherol is alpha-tocopheryl acetate.

7. The composition for the use according to claim 6, consisting of 10-30% alpha-tocopheryl acetate and 70-90% hydrogenated polydecene.

## Patentansprüche

1. Zusammensetzung zur topischen Anwendung zur Verwendung bei der Prävention oder Behandlung von allergischer Rhinitis, bestehend aus
einem Ester von alpha-Tocopherol, gewählt aus der Gruppe, bestehend aus alpha-Tocopherylacetat, -n-proprionat und -linoleat, und
einem ölischen Vehikel, gewählt aus der Gruppe, bestehend aus
hydriertem Polyisobuten, hydriertem Polydecen,
Mischungen aus hydriertem Polyisobuten und/oder hydriertem Polydecen mit hydrierten Polyolefinen, insbesondere hydrierten C₆-C₁₄-hydrierten Polyolefinen,
Capryl-/Caprin-Triglycerid, Olusöl, Adansonia Digitata Öl, Adansonia Digitata Samenöl, Coco-Caprylat/Caprat, Olivensqualan, Olivensqualen, Sonnenblumen (Heliantus Annus) Samenöl, Coco-Caprylat, Isononyl-Isononanoat, Cyclopentasiloxan und Mischungen hiervon.

2. Zusammensetzung zur Verwendung nach Anspruch 1, wobei die Zusammensetzung 3 bis 80 Gew.-% des Esters von alpha-Tocopherol umfasst, bezogen auf das Gesamtgewicht der Zusammensetzung.

3. Zusammensetzung zur Verwendung nach Anspruch 1, wobei die Zusammensetzung 5 bis 40, vorzugsweise 10 bis 30 Gew.-% des Esters von alpha-Tocopherol umfasst, bezogen auf das Gesamtgewicht der Zusammensetzung.

4. Zusammensetzung zur Verwendung nach irgendeinem der Ansprüche 1 bis 3, wobei der Ester von alpha-Tocopherol alpha-Tocopherylacetat ist.

5. Zusammensetzung zur Verwendung nach irgendeinem der Ansprüche 1 bis 4, wobei das ölische Vehikel aus hydriertem Polydecen besteht.

6. Zusammensetzung zur Verwendung nach Anspruch 5, wobei der Ester von alpha-Tocopherol alpha-Tocopherylacetat ist.

7. Zusammensetzung zur Verwendung nach Anspruch 6, bestehend aus 10-30 % alpha-Tocopherylacetat und 70-90 % hydriertem Polydecen.

## Revendications

1. Une composition pour application topique, pour son utilisation dans la prévention ou le traitement de la rhinite allergique, consistant en
un ester d'alpha-tocophérol sélectionné dans le groupe consistant en acétate, n-propionate et linoléate d'alpha-tocophéryle, et
un véhicule huileux sélectionné dans le groupe consistant en
polyisobutène hydrogéné, polydécène hydrogéné,
mélanges de polyisobutène hydrogéné et/ou polydécène hydrogéné avec des polyoléfines hydrogénées, en particulier des polyoléfines hydrogénées en C₆-C₁₄, triglycérides capryliques/capriques, huile végétale, huile d'Adansonia digitata, huile de graines d'Adansonia digitata, caprylate/caprate de noix de coco, squalane d'olive, squalène d'olive, huile de graines de tournesol (Heliantus annuus), caprylate de noix de coco, isononanoate d'isononyle, cyclopentasiloxane, et des mélanges de ceux-ci.

2. La composition pour son utilisation selon la revendication 1, dans laquelle ladite composition comprend de 3 % à 80 % en poids dudit ester d'alpha-tocophérol sur le poids total de la composition.

3. La composition pour son utilisation selon la revendication 1, dans laquelle ladite composition comprend de 5 % à 40 %, de préférence de 10 % à 30 % en poids dudit ester d'alpha tocophérol sur le poids total de la composition.

4. La composition pour son utilisation selon l'une quelconque des revendications 1 à 3, dans laquelle ledit ester d'alpha tocophérol est l'acétate d'alpha-tocophéryle.

5. La composition pour son utilisation selon l'une quelconque des revendications 1 à 4, dans laquelle ledit véhicule huileux consiste en le polydécène hydrogéné.

6. La composition pour son utilisation selon la revendication 5, dans laquelle ledit ester d'alpha tocophérol est l'acétate d'alpha-tocophéryle.

7. La composition pour son utilisation selon la revendication 6, consistant en 10 à 30 % d'acétate d'alpha-tocophéryle et 70 à 90 % de polydécène hydrogéné.
